(19) Europäisches Patentamt
European Patent Office
Office européen des brevets

(11) Veröffentlichungsnummer: **0 019 845**
**B1**

# (12) EUROPÄISCHE PATENTSCHRIFT

(45) Veröffentlichungstag der Patentschrift: **08.09.82**

(51) Int. Cl.³: **C 07 C 43/21,** C 07 C 41/16, C 07 C 29/20

(21) Anmeldenummer: **80102822.6**

(22) Anmeldetag: **21.05.80**

(54) **Ethylether des Isocamphyl-guajakols, Verfahren zu ihrer Herstellung und ihre Verwendung zur Herstellung von 3-(Isocamphyl-(5))-cyclohexanol.**

(30) Priorität: **25.05.79 DE 2921139**

(43) Veröffentlichungstag der Anmeldung: **10.12.80 Patentblatt 80/25**

(45) Bekanntmachung des Hinweises auf die Patenterteilung: **08.09.82 Patentblatt 82/36**

(84) Benannte Vertragsstaaten: **CH DE FR GB IT LI NL**

(56) Entgegenhaltungen:
**DE-A1-2 707 340**
**DE-B2-1 688 427**
**US-A-3 833 671**

(73) Patentinhaber: **HAARMANN & REIMER GMBH, Postfach 138, D-3450 Holzminden (DE)**

(72) Erfinder: **Bauer, Kurt, Dr., Corveyblick 41, D-3450 Holzminden (DE)**
Erfinder: **Lange, Gerd-Karl, Dr., Am Hungerborn 4, D-3450 Holzminden (DE)**

(74) Vertreter: **Dill, Erwin, Dr. et al, c/o BAYER AG Zentralbereich Patente Marken und Lizenzen Bayerwerk, D-5090 Leverkusen 1 (DE)**

**EP 0 019 845 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Ethylether des Isocamphyl-guajakols, Verfahren zu ihrer Herstellung und ihre Verwendung zur Herstellung von 3-(Isocamphyl-(5))-cyclohexanol

Die Erfindung betrifft [Isocamphyl-(5)]-guajacylether der Formel

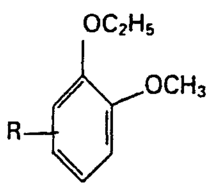

(I)

in der

R für einen in 6- oder 4-Stellung zur Ethoxygruppe befindlichen Isocamphyl-(5)-Rest steht.

Die Erfindung betrifft ferner ein Verfahren zur Herstellung der Ether der Formel (I). Das Verfahren ist dadurch gekennzeichnet, dass man 6- oder 4-[Isocamphyl-(5)]-guajakol in Form ihrer Alkalisalze mit Ethylhalogenid oder Diethylsulfat alkyliert.

Ausserdem betrifft die Erfindung die Verwendung der Ether der Formel (I) bzw. ihrer Gemische zur Herstellung von 3-[Isocamphyl-(5)]-cyclohexanol.

Von den erfindungsgemässen Ethern der Formel (I), dem 6- und 4-[Isocamphyl-(5)]-guajacyl-ethylether, ist der 4-[Isocamphyl-(5)]-guajacyl-ethylether bevorzugt.

Zur Herstellung der erfindungsgemässen Ether der Formel (I) werden 6- oder 4-[Isocamphyl-(5)]-guajakol oder Gemische der beiden Verbindungen mit Alkalihydroxid, insbesondere Natrium- oder Kaliumhydroxyd, mit Alkalialkoholat, insbesondere Natrium- oder Kaliumethylat, oder mit Alkalicarbonaten, insbesondere Kaliumcarbonat, in die Alkaliphenolate überführt. Die Alkylierung erfolgt vorzugsweise bei Verwendung von Alkalihydroxid in wässriger Lösung mit Diethylsulfat, bei Verwendung von Alkalialkoholat in dem entsprechenden Alkohol mit Ethyljodid, Ethylbromid oder Diethylsulfat oder bei Verwendung von Kaliumcarbonat in Aceton mit Diethylsulfat.

Als Gemische aus 6- und 4-[Isocamphyl-(5)]-guajakol kann man auch die Reaktionsprodukte einsetzen, wie sie z.B. bei der Kondensation von Camphen und Guajakol anfallen. Diese Reaktionsprodukte bestehen überwiegend aus den beiden genannten Guajakolen. Die in den Reaktionsprodukten weiterhin enthaltenen Verbindungen, stören nicht bei der Veretherungsreaktion.

Als besonders vorteilhaft hat sich die Umsetzung von 6- und 4-[Isocamphyl-(5)]-guajakol mit Diethylsulfat in Gegenwart von Kaliumcarbonat in Aceton erwiesen.

Isocamphyl-(5)-guajakol und Alkalihydroxid bzw. alkoholat werden vorteilhaft in einem Molverhältnis von 1:1-5, vorzugsweise 1:1-3 angewendet. Das Alkylierungsmittel wird in einer Menge von 1,2 bis 4 Mol, vorzugsweise 1,25 bis 3 Mol pro Mol Isocamphyl-(5)-guajakol eingesetzt.

Die Alkylierung wird vorteilhaft bei 0 bis 80° C, vorzugsweise 30 bis 60° C, vorgenommen.

Zur Weiterverarbeitung zum 3-[Isocamphyl-(5)]-cyclohexanol werden die erfindungsgemässen Ether der Formel (I) zunächst partiell zum 5- bzw. 3-[Isocamphyl-(5)]-2-ethoxy-phenol gespalten.

Die partielle Etherspaltung kann mit Alkalialkoholat in Alkohol, z.B. Natrium- oder Kaliummethylat in Methanol oder Natrium- oder Kaliumethylat in Ethanol, bei 130 bis 200° C, vorzugsweise 150 bis 180° C, im Autoklaven durchgeführt werden.

Bevorzugt ist aber die partielle Etherspaltung mit einem Grignard-Reagenz wie Methylmagnesiumbromid oder -jodid bei Temperaturen von 100 bis 160° C, vorzugsweise 120 bis 140° C.

Das bei der partiellen Etherspaltung der erfindungsgemässen Ether der allgemeinen Formel (I) anfallende 5- bzw. 3-[Isocamphyl-(5)]-2-ethoxy-phenol wird dann mit Raney-Nickel bei einer Temperatur von 180 bis 210° C und einem Druck von 130 bis 200 bar mit Wasserstoff zu 3-[Isocamphyl-(5)]-cyclohexanol hydriert.

Die erfindungsgemässen Ether der Formel (I) sind wertvolle Zwischenprodukte zur Herstellung von 3-[Isocamphyl-(5)]-cyclohexanol, einem wichtigen Inhaltsstoff des Sandelkörpers. Mit ihrer Hilfe lässt sich 3-[Isocamphyl-(5)]-cyclohexanol in wesentlich wirtschaftlicherer Weise als nach den bisher bekannten Verfahren herstellen.

Die in C. A. *68,* (1968), 39823 z als Zwischenprodukte für die Herstellung des 3-[Isocamphyl-(5)]-cyclohexanols vorgeschlagene 2,4-Dinitrophenylether des 6- und 4-[Isocamphyl-(5)]-guajakols liefern 3-[Isocamphyl-(5)]-phenol, die letzte Vorstufe bei der Herstellung des 3-[Isocamphyl-(5)]-cyclohexanols, nur in etwa 50%iger Ausbeute, bezogen auf 4-[Isocamphyl-(5)]-guajakol. Mit Hilfe der erfindungsgemässen Ether der Formel (I) wird dagegen 3-[Isocamphyl-(5)]-cyclohexanol in bis zu 85%iger Ausbeute, bezogen auf 4-[Isocamphyl-(5)]-guajakol, erhalten.

Ausserdem sind die erfindungsgemässen Ether unvergleichlich viel billiger als die 2,4-Dinitrophenylether, zu deren Herstellung das schwierig zugängliche und teure 2,4-Dinitrofluorbenzol benötigt wird.

Das in der DE-AS-12 23 482 vorgeschlagene Verfahren zur Herstellung von 3-[Isocamphyl-(5)]-cyclohexanol ist noch wesentlich unwirtschaftlicher als das in C. A. *68,* (1968), 39823 z beschriebene Verfahren. Es geht von Terpenylphenolen aus, die in einer komplizierten Vielstufen-Reaktion über Terpenyl-methylketone in 3-[Isocamphyl-(5)]-cyclohexen-(2)-on-(1) überführt werden, was dann zum 3-[Isocamphyl-(5)]-cyclohexanol hydriert wird. Die Ausbeute beträgt bei diesem Verfahren noch nicht einmal 20%, bezogen auf das Ausgangs-terpenylphenol.

Das in der DE-AS-27 07 340 beschriebene Verfahren zur Herstellung von Gemischen mit Sandelholzaroma, bei dem Camphen mit Brenzcatechin umgesetzt und das erhaltene Gemisch dann über ein nichtriechendes Gemisch aus Diolverbindungen zum Endprodukt hydriert wird, verläuft nur in mässigen Ausbeuten. Im Endprodukt selbst

stellt das 3-[Isocamphyl-(5)]-cyclohexanol nur einen geringen Teil dar, da weder die Umsetzung von Camphen mit Brenzcatechin noch die Entfernung einer Hydroxygruppe spezifisch erfolgt.

*Beispiel 1*

Die Lösung von 598g (2,3 Mol) 4-[Isocamphyl-(5)]-guajakol in 2,3l Aceton wird mit 955g (6,93 Mol) Kaliumcarbonat versetzt und auf 50° C erwärmt. Dann werden innerhalb einer Stunde 1150g (7,47 Mol) Diethylsulfat zugetropft und nach beendeter Zugabe 8 Stunden unter Rückfluss gekocht. Anschliessend wird abgekühlt, das Kaliumsalz abfiltriert und das Lösungsmittel abdestilliert. Der Rückstand wird im Vakuum destilliert.

Ausbeute: 622g (= 94% der Theorie) 4-[Isocamphyl-(5)]-guajacyl-ehtylether.
Kp.: 150 bis 152° C/0,35 Torr.

Werden anstelle des 4-[Isocamphyl-(5)]-guajakols 598g eines Terpenylguajakol-Gemisches, erhalten durch Umsetzung von Camphen mit Guajakol in Gegenwart eines Friedel-Craft-Katalysators, eingesetzt, erhält man ein bei 163 bis 240° C/l Torr überdestillierendes Terpenylguajacyl-ethylether-Gemisch in einer Ausbeute von 525g (= 79,3% der Theorie).

*Beispiel 2*

Zu einer frisch bereiteten Lösung von Methylmagnesiumjodid in Diethylether, hergestellt aus 35g (1,44 Mol) Magnesium und 215g (1,5 Mol) Methyljodid in 400ml Diethylether, wird eine Lösung von 396g (1,375 Mol) 4-[Isocamphyl-(5)]-guajacyl-ethylether in 500ml Xylol zugetropft. Dann wird der Diethylether abdestilliert. Weitere 500ml Xylol werden zugesetzt, um das Reaktionsgemisch in Lösung zu halten und 8 Stunden unter Rückfluss gekocht. Nach dem Abkühlen wird unter Eiskühlung mit 1l 25%iger Schwefelsäure versetzt. Nach Zugabe von 1l Toluol wird gut durchgemischt und die wässrige Phase abgetrennt. Die organische Phase wird neutral gewaschen und die Lösungsmittel abgezogen. Der verbleibende Rückstand wird destilliert.

Ausbeute: 360g (= 95,5% der Theorie) 5-[Isocamphyl-(5)]-2-ethoxy-phenol.
Kp.: 147 bis 150° C/0,4 Torr.

Setzt man anstelle des 4-[Isocamphyl-(5)]-guajacyl-ethylethers 396g des Terpenyl-guajacyl-ethylether-Gemisches ein, erhält man 361,5g (= 95,9% der Theorie) einer Terpenyl-ethoxy-phenol-Gemisches, das bei 144 bis 173° C/0,7 Torr überdestilliert.

*Beispiel 3*

300g (1,09 Mol) 5-[Isocamphyl-(5)]-2-ethoxy-phenol werden in Gegenwart von 30g Raney-Nickel unter einem Wasserstoffdruck von 155 Atmosphären bei 200° C hydriert. Nach 20 Stunden ist die Wasserstoffaufnahme beendet. Nach Zugabe von 1l Tetrachlorkohlenstoff wird der Katalysator abfiltriert und das Filtrat vom Lösungsmittel befreit. Der Rückstand wird destilliert.

Ausbeute: 243g (= 94,2% der Theorie) 3-[Isocamphyl-(5)]-cyclohexanol (cis-trans-Isomeren-Gemisch).
Kp.: 130 bis 135° C/0,2 Torr.

Werden anstelle des 5-[Isocamphyl-(5)]-2-ethoxy-phenols 300g des Terpenyl-ethoxy-phenol-Gemisches in die Hydrierung eingesetzt, erhält man 244g (= 94,6% der Theorie) eines Terpenylcyclohexanol-Gemisches, das bei 122 bis 171° C/1 Torr überdestilliert.

**Patentansprüche**

1. [Isocamphyl-(5)]-guajacyl-ether der Formel

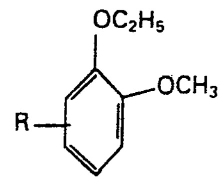

in der
R für einen in 6- oder 4-Stellung zur Ethoxygruppe befindlichen Isocamphyl-(5)-Rest steht.

2. 4-[Isocamphyl-(5)]-guajacyl-ethylether.

3. Verfahren zur Herstellung von [Isocamphyl-(5)]-guajacyl-ether der Formel

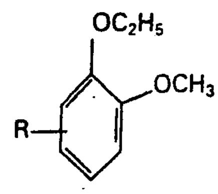

in der
R für ein in 6- oder 4-Stellung zur Ethoxygruppe befindlicher Isocamphyl-(5)-Rest steht, dadurch gekennzeichnet, dass man 6- oder 4-[Isocamphyl-(5)]-guajakol in Form ihrer Alkalisalze mit Ethylhalogenid oder Diethylsulfat alkyliert.

4. Verwendung der [Isocamphyl-(5)]-guajacyl-ether gemäss Anspruch 1 und 2 zur Herstellung von 3-[Isocamphyl-(5)]-cyclohexanol.

**Claims**

1. (Isocamph-5-yl)-guaiacyl ethers of the formula

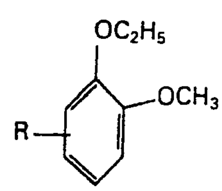

in which
R represents an isocamph-5-yl radical in the 6- or 4-position relative to the ethoxy group.

2. 4-(Isocamph-5-yl)-guaiacyl ethyl ether.

3. Process for the preparation of (isocamph-5-yl)-guaiacyl ethers of the formula

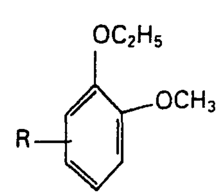

in which

R represents an isocamph-5-yl radical in the 6- or 4-position relative to the ethoxy group, characterised in that 6- or 4-(isocamph-5-yl)-guaiacol in the form of its alkali metal salts, is alkylated with an ethyl halide or with diethyl sulphate.

4. Use of the (isocamph-5-yl)-guajacyl ethers according to Claim 1 and 2 for the preparation of 3-(isocamph-5-yl)-cyclohexanol.

**Revendications**

1. Ethers [isocamphyl-(5)]-gaïacyliques de formule

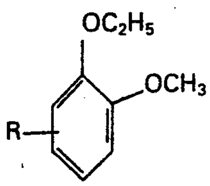

dans laquelle

R représente un reste isocamphyle-(5) en position 6 ou 4 par rapport au groupe éthoxy.

2. L'oxyde de 4-[isocamphyl-(5)]-gaïacyle et d'éthyle.

3. Procédé de préparation des éthers [isocamphyl-(5)]-gaïacyliques de formule

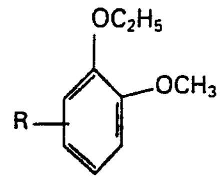

dans laquelle

R représente un reste isocamphyle-(5) en position 6 ou 4 par rapport au groupe éthoxy, caractérisé en ce que l'on alkyle le 6- ou le 4-[isocamphyl-(5)]-gaïacol à l'état de sel alcalin par un halogénure d'éthyle ou le sulfate de diéthyle.

4. Utilisation des éthers [isocamphyl-(5)]-gaïacyliques selon les revendications 1 et 2 pour la préparation du 3-[isocamphyl-(5)]-cyclohexanol.